# EUROPEAN PATENT APPLICATION

(11) **EP 3 075 339 A1**
(43) Date of publication of application: **05.10.2016**
(21) Application number: 14866674.6
(22) Date of filing: 14.11.2014
(51) Int. Cl.: A61B 18/14

(54) **ELECTRODE APPARATUS FOR RADIOFREQUENCY ABLATION**

(30) Priority: 27.11.2013 KR 20130145365
(71) Applicant: Starmed Co., Ltd., Ilsandong-gu, Goyang-si Gyeonggi-do 410-722 (KR)
(72) Inventor: SHIN, Kyung Hoon, Gimpo-si (KR); KIM, Dong Un, Gimpo-si (KR); SHIN, Kyong Min, Yangpyeong-gun (KR)
(74) Representative: Burger, Markus
(86) International application number: PCT/KR2014/010984
(87) International publication number: WO 2015/080411

(57) **Abstract**

The present invention relates to an electrode device for radiofrequency ablation, which can easily be used for computed tomography (CT)-guided radiofrequency ablation (RFA) treatment. An embodiment of the present invention provides an electrode device for radiofrequency ablation, which comprises an electrode needle provided in front of a grip and is configured to cauterize and necrotize a diseased site by radiofrequency heat generated from the electrode needle, the electrode device comprising a bendable tube which is provided between the grip and the electrode needle, is bendable at a predetermined angle, and is capable of being maintained in a bent state.

## Description

### [Technical Field]

The present invention relates to an electrode device for radiofrequency ablation, which is configured to cauterize and necrotize a diseased site such as cancer tissue in a body organ by heating the diseased site with radiofrequency energy, and more particularly, to an electrode device for radiofrequency ablation, which is easily used for computed tomography (CT)-guided radiofrequency ablation (RFA) treatment.

### [Background Art]

In general, cancer tissue created in a body organ, for example, an organ such as the liver, is treated by non-operative methods or surgical operations.

However, the surgical operation has problems in that, because a body portion corresponding to a diseased site should be excised, the excised portion is very large so that a large scar remains, and in that a long convalescence period is required.

For this reason, non-operative methods, for example, transarterial chemoembolization, percutaneous ethanol injection therapy, systemic anticancer chemotherapy, local thermotherapy and the like, have recently been used. Among these methods, local thermal therapy is known to be most effective in terms of short-term treatment results or improved long-term survival rates.

The local thermotherapy methods include radiofrequency ablation, microwave cauterization, laser cauterization, etc. Among them, radiofrequency ablation has been most effectively used.

As used herein, the term "radiofrequency ablation" refers to a therapeutic method in which only cancer tissue created in a body organ, for example, the liver, is cauterized and necrotized by radiofrequency heating without being excised.

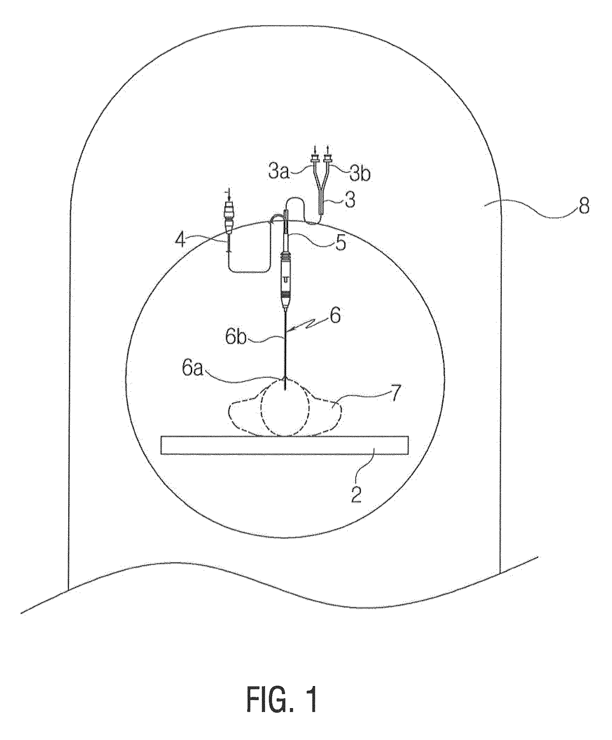

FIG. 1 shows an example of an electrode device for radiofrequency ablation. As shown therein, an electrode needle 6 is provided in front of a grip 5. In the rear of the grip 5, there is disposed an electrode line 4 configured to supply radiofrequency energy to the electrode needle 6 and a cooling line 3 configured to circulate and supply cooling water into the electrode needle 6 so as to prevent the electrode needle from being melted by radiofrequency heating.

Herein, the electrode needle 6 generally has a current applying portion 6a formed in a portion of the tip thereof and an insulated portion 6b formed in the remaining portion. The current applying portion 6a of the electrode needle 6 may have various lengths such that a suitable size can be selected according to the size of a diseased site in a body organ. Namely, in order to secure treatment, the current applying portion 6a is formed to have a length longer than at least a length that penetrates the diseased site so that radiofrequency heat applied by the current passing portion 6a will cauterize a site larger than the diseased site.

However, in the case in which a computed tomography (CT) system is used to accurately position the electrode needle at the diseased site for radiofrequency ablation treatment (CT-guided RFA treatment), a patient's body 7 is located in the ring body 8 of the CT system in a state in which it is laid on a bed 2 as shown in FIG. 1.

In this case, if the length of the electrode device comprising the electrode needle 6 and the grip 5 is longer than the distance between the body 7 and the inner circumferential surface of the ring body 8, there is a problem in that the electrode needle 6, grip 5, electrode line 4 or cooling line 3 of the electrode device is interfered with by the ring body 8, making the treatment inconvenient.

### [Disclosure]

### [Technical Problem]

The present invention has been made in order to solve the above-described problems, and an embodiment of the present invention relates to an electrode device for radiofrequency ablation, in which a bendable tube, which can be deformable to be bent and can be maintained in a bent state, is provided between an electrode needle and a grip.

### [Technical Solution]

In accordance with a preferred embodiment of the present invention, there is provided an electrode device for radiofrequency ablation, which comprises an electrode needle provided in front of a grip and is configured to cauterize and necrotize a diseased site by radiofrequency heat generated from the electrode needle, the electrode device comprising a bendable tube which is provided between the grip and the electrode needle, is bendable at a predetermined angle, and is capable of being maintained in a bent state.

In the electrode device for radiofrequency ablation according to the embodiment of the present invention, the bendable tube comprises: an inner tube which is made of a soft material and one end of which is coupled to the tip of the grip; and an outer corrugated tube, one end of which is coupled to the tip of the grip and which is configured to receive the inner tube therein.

The electrode device for radiofrequency ablation according to the embodiment of the present invention may further comprise a connection tube, one end of which is coupled so as to cover the outer circumferential surface of the other end of the outer tube and the other end of which is coupled so as to cover one end of the electrode needle.

The electrode device for radiofrequency ablation according to an embodiment of the present invention may further comprise a guide tube, one end of which is inserted into the other end of the inner tube and the other end of which extends to the tip of the electrode needle.

In the electrode device for radiofrequency ablation according to the embodiment of the present invention, the grip of the electrode device for radiofrequency ablation may comprise: a first block to which a cooling water supply line and a cooling water discharge line are connected and in which a first cooling water supply channel and a first cooling water discharge channel are separately formed; a second block which is connected to one side of the first block and in which a second cooling water supply channel communicating with the first cooling water supply channel and the inner tube and a second cooling water discharge channel communicating with the first cooling water discharge channel are separately formed; and a third block which is coupled to one side of the second block and into which one end of each of the outer tube and the inner tube is inserted.

In the electrode device for radiofrequency ablation according to the embodiment of the present invention, the grip is configured such that a first protruding portion in the first block is inserted into and coupled to a second depressed portion formed at one side of the second block and such that cooling water introduced into the second block through the first cooling water supply channel flows to the second cooling water supply channel through a first space portion which is formed when the first protruding portion is coupled to the second depressed portion.

In the electrode device for radiofrequency ablation according to the embodiment of the present invention, the grip is configured such that a second protruding portion in the second block is inserted into and coupled to a third depressed portion formed at one side of the third block and such that cooling water introduced into the third block through a gap formed between the inner tube and the outer tube flows to the second cooling water discharge channel through a second space portion formed between the second protruding portion and the third depressed portion.

In the electrode device for radiofrequency ablation according to the embodiment of the present invention, the grip is configured such that one end of the outer tube is inserted into a through-hole formed in the center of the third block in the lengthwise direction, and one end of the inner tube is inserted into the second cooling water supply channel through the through-hole.

### [Advantageous Effects]

The electrode device for radiofrequency ablation according to the embodiment of the present invention comprises the bendable tube provided between the grip and the electrode needle, and thus the grip can be bent at a certain angle with respect to the electrode needle and maintained in the bent state. Thus, it is possible to solve the problem in that one side of the electrode device is interfered with by the ring body of the CT system during CT-guided radiofrequency ablation treatment, making the treatment inconvenient.

In addition, the grip of the electrode device for radiofrequency ablation according to the embodiment of the present invention is formed of three blocks (first, second and third blocks), and a cooling water supply channel and a cooling water discharge channel are separately formed in the blocks so that cooling water will be circulated to the tip of the electrode needle. Thus, it is possible to prevent the electrode needle from being broken by overheat.

### [Description of Drawings]

FIG. 1 shows the configuration of an electrode device for radiofrequency ablation according to a conventional art.
FIG. 2 shows the configuration of an electrode device for radiofrequency ablation according to an embodiment of the present invention.
FIG 3 is an enlarged sectional view of portion "A" shown in FIG. 2.
FIG 4 is an enlarged sectional view of portion "B" shown in FIG. 2.
FIG 5 is an enlarged sectional view of portion "C" shown FIG. 2.
FIG. 6 shows a state in which an electrode device for radiofrequency ablation according to an embodiment of the present invention is used.

### Description of reference numerals used in the drawings

100: electrode device; 200: grip;
210: first block; 213: first cooling water supply channel;
214: first cooling water discharge channel;
216: line insertion hole; 220: second block;
223: second cooling water supply channel;
224: second cooling water discharge channel;
230: third block; 233: through-hole;
300: bendable tube; 310: inner tube;
320: outer tube; 400: guide tube;
500: connection tube; 600: electrode needle.

### [Best Mode]

Hereinafter, an electrode device for radiofrequency ablation according to a preferred embodiment of the present invention will be described with reference to the accompanying drawings. In the drawings, the thickness of lines or the size of constituent elements may be exaggerated for the clarity and convenience of description.

Also, the terms used in the following description are terms defined taking into consideration their functions in the present invention, and may be changed in accordance with the intention of a user or an operator, or a usual practice. Accordingly, definitions of these terms must be based on the entire disclosure of the specification.

In addition, the following embodiment is not construed to limit the scope of the present invention and is merely illustrative of the constituent elements set forth in the claims of the present invention, and the scope of the present invention may encompass embodiments which are included in the technical idea of present invention and which include constituent elements that are replaceable as equivalents of the constituent elements in the claims.

### Embodiment

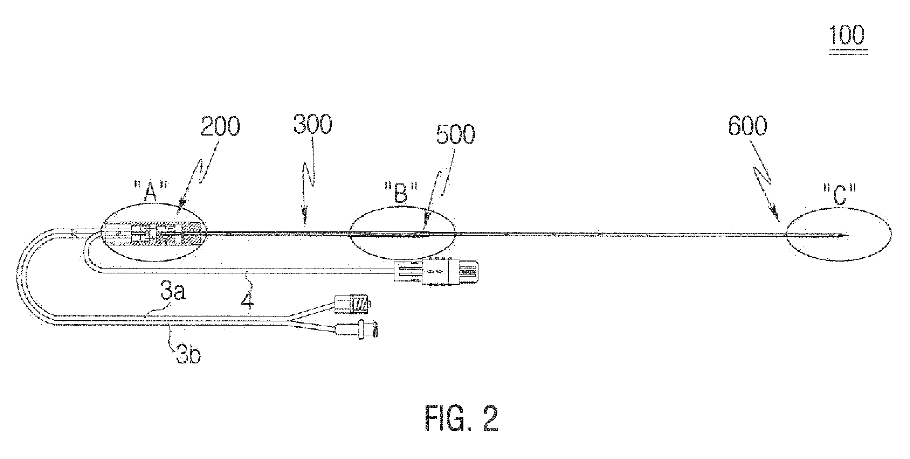

FIG. 2 shows the configuration of an electrode device for radiofrequency ablation according to an embodiment of the present invention.

As shown in FIG. 2, an electrode device 100 for radiofrequency ablation (hereinafter referred to as "electrode device") according to an embodiment of the present invention comprises an electrode needle 600 provided in front of a grip 200, and is a medical device configured to cauterize and necrotize a diseased site by radiofrequency heat generated from the electrode needle 600. The electrode device comprises a bendable tube 300 between the grip 200 and the electrode needle 600.

The bendable tube 300 can be bent at a certain angle, and can be maintained in a bent state unless a further external force is applied thereto. Thus, in CT-guided radiofrequency ablation treatment, one end of the bendable tube 300 may be bent so that the grip 200 can be maintained in a state bent at a certain angle with respect to the electrode needle 600. In this case, the length of the electrode device 100 that extends straight to the outside of the body during radiofrequency ablation treatment is shortened, and thus the electrode device 100 can be moved freely in the space between the ring body 8 of the CT system and the body 7 so that radiofrequency ablation treatment can be easily performed.

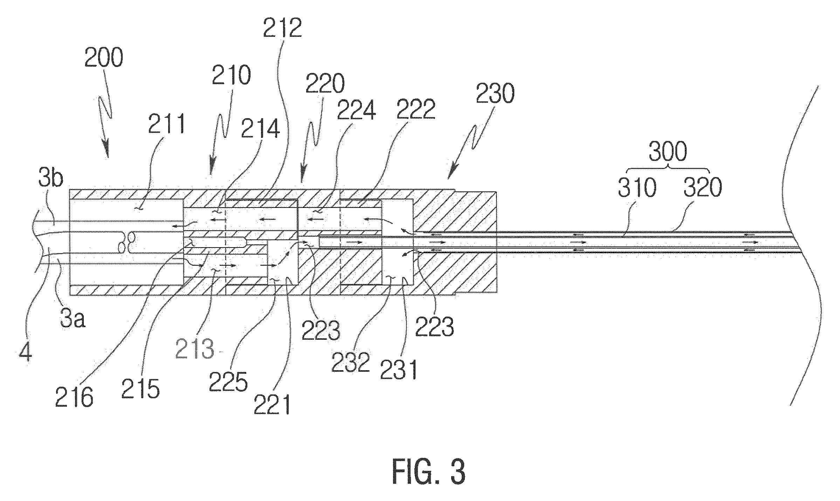

FIG. 3 is an enlarged sectional view of portion "A" shown FIG. 2. The arrows shown in the drawings indicate the flow direction of cooling water.

In an embodiment of the present invention, the grip 200 comprises three blocks having a cylindrical or polygonal column shape and coupled to one another in the lengthwise direction, and a cooling water supply channel and a cooling water discharge channel are separately formed in each o the three blocks.

The first block 210 is located in the grip 200 at a position most far from the tip of the electrode needle 600, and has a first depressed portion 211 formed at the rear end thereof and a first protruding portion 212 formed at the front end thereof. To the rear end of the first block 210, a cooling water supply line 3a, a cooling water discharge line 3b and an electrode line 4 are connected, and each of the lines is inserted into the first depressed portion 211.

In front of the first depressed portion 211, a first cooling water supply channel 213 and a first cooling water discharge channel 214 are formed with a boss portion 215 interposed therebetween, and a line insertion hole 216 is formed in the center of the boss portion 215. Herein, the first cooling water supply channel 213 and the first cooling water discharge channel 214 are formed to extend from the first depressed portion 211 to the end of the first protruding portion 212. Through the line insertion hole 216, a line (not shown) for the electrode line 4 configured to supply radiofrequency energy to the electrode needle 600 extends to one end of the electrode needle 600.

The second block 220 is coupled to one side of the first block 210. At the rear end of the second block 220, a second depressed portion 221 is formed, and at the front end of the second block, a second protruding portion 222 is formed. More specifically, the first protruding portion 212 of the first block 210 is inserted into the second depressed portion 221 of the second block 220, and the outer circumferential surface of the first protruding portion 212 is coupled to the inner circumferential surface of the second depressed portion 221 by a method such as interference fitting or bonding.

In the center of the second block 220, a second cooling water supply channel 223 is formed, and in the outside of the second cooling supply channel 223, a second cooling water discharge channel 224 is separately formed. Herein, the first cooling water discharge channel 214 of the first block 210 and the second cooling water discharge channel 224 of the second block 220 are communicated with each other, and for this purpose, one side of the first protruding portion 212 of the first block 210 is preferably formed to extend to the second cooling water discharge channel 224.

Meanwhile, when the first block 210 is coupled to the second block 220, a first space portion 225 in the second depressed portion 221 of the second block 220 is formed between the first block 210 and the second block 220. Cooling water introduced into the second block 220 through the first cooling water supply channel 213 will flow in the direction of the second cooling water supply channel 223 through the first space portion 225.

The third block 230 is coupled to one side of the second block 230. At the rear end of the third block 230, a third depressed portion 231 is formed. More specifically, the second protruding portion 222 of the second block 220 is inserted into the third depressed portion 231 of the third block 230, and the outer circumferential surface of the second protruding portion 222 is coupled to the inner circumferential portion of the third depressed portion 231 by a method such as interference fitting or bonding. In addition, when the second protruding portion 222 is coupled to the third depressed portion 231, a second space portion 232 is formed between the second block 220 and the third block 230.

In the center of the third block 230, a through-hole 233 is formed in the lengthwise direction, and one end of each of the inner tube 310 and the outer tube 320 is inserted into the through-hole 233. Herein, one end of the inner tube 310 is inserted into and coupled to the second cooling water supply channel 223 of the second block 220, and one end of the outer tube 320 is inserted into and coupled to the through-hole 233.

The inner tube 310 is provided in the outer tube 320, and a certain gap is formed between the outer circumferential surface of the inner tube 310 and the inner circumferential surface of the outer tube 320.

Cooling water is supplied to the first block 210 through the cooling water supply line 3a and introduced into the second block 220 through the fist cooling water supply channel 213 of the first block 210. The cooling water introduced into the second block 220 flows to the second cooling water supply channel 223 through the first space portion 225, passes through the inner tube 310 connected to the second cooling water supply channel 223, and flows to the tip of the electrode needle 600 through a guide tube 400 as described below.

The cooling water supplied to the tip of the electrode needle 600 is circulated again to the cooling water discharge line. Specifically, the cooling water is introduced into the second space portion 232 of the third block 230 through the gap between the guide tube 400 and the electrode needle 600 and the gap between the inner tube 310 and the outer tube 320, and is circulated to the cooling water discharge line 3b through the second cooling discharge channel 224 of the second block 220 and the first cooling water discharge channel 214 of the first block 210.

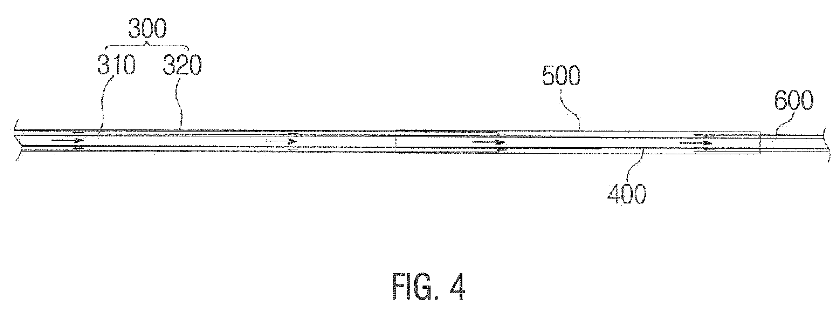

FIG. 4 is an enlarged sectional view of portion "B" shown FIG. 2.

One end of the bendable tube 300 is coupled to the tip of the grip 200. Herein, the bendable tube comprises an inner tube 310 and an outer tube 320 surrounding the outside of the inner tube 310. A certain gap is formed between the outer circumferential surface of the inner tube 310 and the inner circumferential surface of the outer tube 320 so that cooling water that is circulated to the cooling water discharge line 3b will flow in the direction of the grip 200 through the gap.

The other end of the inner tube 310 may protrude from the other end of the outer tube 320 in the direction of the electrode needle 600 by a certain length, and the guide tube 400 is inserted into and coupled to the other end of the inner tube 310. Herein, one end of the guide tube 400 is inserted into the other end of the inner tube 310, and the other end of the guide tube 400 is inserted into the electrode needle 600 and extends to the tip of the electrode needle 600. Thus, cooling water that flows through the inner tube 310 will flow to the tip of the electrode needle 600 through the guide tube 400.

Meanwhile, one end of a connection tube 500 is coupled to the outer circumferential surface of the other end of the outer tube 320, and one end of the electrode needle 600 is coupled to the inside of the other end of the connection tube 500. Namely, the connection tube 500 is connected to the other end of the outer tube 320 so as to cover the outer circumferential surface of one end of the electrode needle 600.

Herein, cooling water is circulated in the direction of the grip 200 through the gap between the guide tube 400 and the electrode needle 600, and the cooling water introduced into the grip 200 after sequential passage through the gap between the guide tube 400 and the connection tube 500, the gap between the inner tube 310 and the connection tube 500 and the gap between the inner tube 310 and the outer tube 320 is discharged through the cooling water discharge line 3b.

Meanwhile, the electrode device 100 according to the embodiment of the present invention is characterized in that the grip 200 is deformable to be bent at a certain angle with respect to the electrode needle 600 and can be maintained in a bent state.

For this, the inner tube 310 and the outer tube 320, which are disposed between the grip 200 and the electrode needle 600, is preferably made of an electrically deformable soft material such as synthetic resin, and the outer tube 320 is preferably formed of a corrugated tube so that it can be maintained in a bent state unless a further external force is applied thereto. In addition, the outer tube 320 preferably has a certain hardness so that the depth of insertion of the electrode needle 600 can be controlled by manipulating the grip 200 in a state in which the grip 200 is bent at a certain angle with respect to the electrode needle 600.

Meanwhile, the connection tube 500 is preferably made of an elastically bendable synthetic resin having a certain hardness, for example, polyether ether ketone (PEEK).

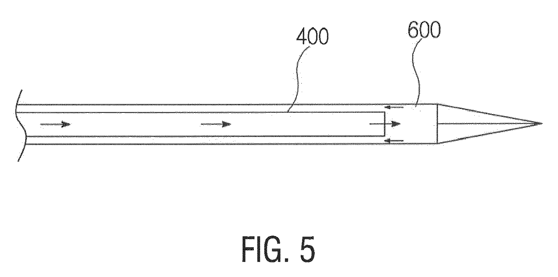

FIG. 5 is an enlarged sectional view of portion "C" shown in FIG. 2.

The guide tube 400 as described above is inserted into the electrode needle 600 and extends to the tip of the electrode needle 600. Cooling water supplied to the tip of the electrode needle 600 through the guide tube 400 is circulated in the direction of the grip 200 through the gap between the outer circumferential surface of the guide tube 400 and the inner circumferential surface of the electrode needle 600.

Herein, the electrode needle 600 is configured to emit radiofrequency energy in a state inserted into a tissue of a diseased site to thereby cauterize and necrotize the surrounding tissue, and is preferably made of a metal material such as stainless steel, which is harmless to the human body, is rustproof, can pass an electric current therethrough.

The electrode line 4 configured to supply radiofrequency energy to the electrode needle 600 may extend in the direction of the electrode needle 600 through the line insertion hole 216 of the first block and the gap between the outer tube 320 and the inner tube 310, and may be connected to one side of the electrode needle 600. In addition, a portion of the electrode needle 600 may also be insulated so that the insulated portion will not generate heat, and thus a tissue portion that is cauterized and a tissue portion that is not cauterized can be distinguished from each other according to the depth of insertion of the electrode needle 600 into tissue during radiofrequency ablation treatment.

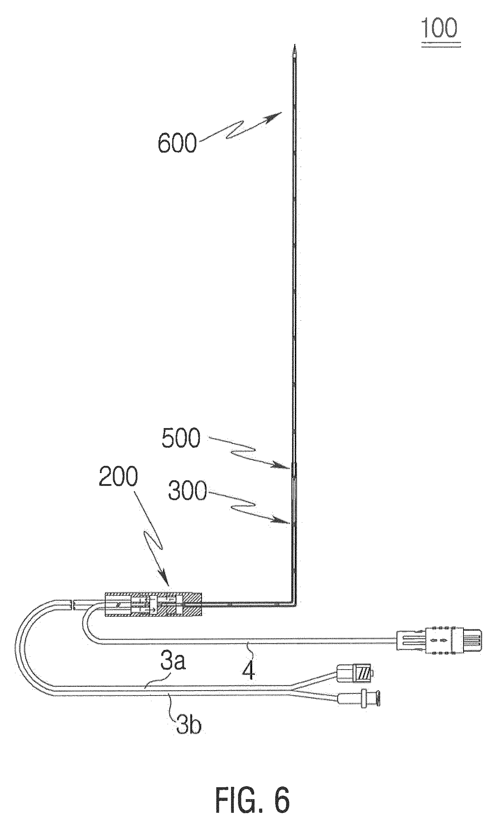

FIG. 6 shows a state in which the electrode device for radiofrequency ablation according to the embodiment of the present invention is used.

In the electrode device 100 for radiofrequency ablation according to the embodiment of the present invention, the inner tube 310 and the outer tube 320, which are disposed between the grip 200 and the electrode needle 600, are made of an elastically bendable material. Thus, when an operation for CT-guided radiofrequency treatment is inconvenient because the length of the electrode device 100 is longer than the distance between the body 7 and the ring body 8 of the CT system, the grip 200 can be bent at a certain angle with respect to the electrode needle 600 as shown in FIG. 6 to shorten the length of the electrode device 100, so that the CT-guided radiofrequency treatment can be conveniently performed.

Herein, the outer tube 320 is preferably made of a material having a certain hardness so that the grip 200 can be bent at a certain angle with respect to the electrode needle 600 and maintained in the bent state. In addition, the outer tube 320 is preferably formed of a corrugated tube.

## Claims

1. An electrode device for radiofrequency ablation, which comprises an electrode needle 600 provided in front of a grip 200 and is configured to cauterize and necrotize a diseased site by radiofrequency heat generated from the electrode needle 600, the electrode device comprising a bendable tube 300 which is provided between the grip 200 and the electrode needle 600, is bendable at a predetermined angle, and is capable of being maintained in a bent state.

2. The electrode device for radiofrequency ablation according to claim 1, wherein the bendable tube 300 comprises:
an inner tube 310 which is made of a soft material and one end of which is coupled to a tip of the grip 200; and
an outer corrugated tube 320, one end of which is coupled to the tip of the grip 200 and which is configured to receive the inner tube 310 therein.

3. The electrode device for radiofrequency ablation according to claim 2, further comprising a connection tube 500, one end of which is coupled so as to cover an outer circumferential surface of the other end of the outer tube 320 and the other end of which is coupled so as to cover one end of the electrode needle 600.

4. The electrode device for radiofrequency ablation according to claim 2, further comprising a guide tube 400, one end of which is inserted into the other end of the inner tube 310 and the other end of which extends to a tip of the electrode needle 600.

5. The electrode device for radiofrequency ablation according to claim 2, wherein the grip 200 comprises:
a first block 210 to which a cooling water supply line 3a and a cooling water discharge line 3b are connected and in which a first cooling water supply channel 213 and a first cooling water discharge channel 214 are separately formed;
a second block 220 which is connected to one side of the first block 210 and in which a second cooling water supply channel 223 communicating with the first cooling water supply channel 213 and the inner tube 310 and a second cooling water discharge channel 224 communicating with the first cooling water discharge channel 214 are separately formed; and
a third block 230 which is coupled to one side of the second block 220 and into which one end of each of the outer tube 320 and the inner tube 310 is inserted.

6. The electrode device for radiofrequency ablation according to claim 5, wherein a first protruding portion 212 in the first block 210 is inserted into and coupled to a second depressed portion 221 formed at one side of the second block 220, and cooling water introduced into the second block 220 through the first cooling water supply channel 213 flows to the second cooling water supply channel 223 through a first space portion 225 which is formed when the first protruding portion 212 is coupled to the second depressed portion 221.

7. The electrode device for radiofrequency ablation according to claim 5, wherein a second protruding portion 222 in the second block 220 is inserted into and coupled to a third depressed portion 231 formed at one side of the third block 230, and cooling water introduced into the third block 230 through a gap formed between the inner tube 310 and the outer tube 320 flows to the second cooling water discharge channel 224 through a second space portion 232 formed between the second protruding portion 222 and the third depressed portion 231.

8. The electrode device for radiofrequency ablation according to claim 5, wherein one end of the outer tube 320 is inserted into a through-hole 233 formed in a center of the third block 230 in a lengthwise direction, and one end of the inner tube 310 is inserted into the second cooling water supply channel 223 through the through-hole 233.
